# EUROPEAN PATENT APPLICATION

(11) **EP 4 144 748 A1**
(43) Date of publication of application: **08.03.2023**
(21) Application number: 21796576.3
(22) Date of filing: 29.04.2021
(51) Int. Cl.: C07K 1/113, C07K 14/565, C12N 15/85, C07K 14/715, A61K 38/21, A61P 35/00, A61P 31/12, A61P 25/00

(54) **HUMAN INTERFERON-BETA VARIANT WITH DOUBLE MUTATION AND METHOD FOR IMPROVING STABILITY OF HUMAN INTERFERON-BETA VARIANT**

(30) Priority: 29.04.2020 KR 20200052513; 29.04.2020 KR 20200052913
(71) Applicant: Abion Inc., Seoul 08394 (KR)
(72) Inventor: CHOI, Jun Young, Seoul 08394 (KR); KIM, Na Young, Seoul 08394 (KR); SON, Won Rak, Seoul 08394 (KR); HONG, Sung Hyun, Seoul 08394 (KR); LEE, Yong Jin, Seoul 08394 (KR); LEE, Sae Hyung, Seoul 08394 (KR)
(74) Representative: Schwarz & Partner Patentanwälte GmbH
(86) International application number: PCT/KR2021/005487
(87) International publication number: WO 2021/221485

(57) **Abstract**

The present invention relates to a human interferon-beta variant with double mutation and a method for improving stability of a human interferon-beta variant and, more specifically, to a human interferon-beta variant including an amino acid sequence having serine substituted for the 17th amino acid cysteine of human interferon-beta and threonine substituted for the 27th amino acid arginine of the human interferon-beta, and a method for improving stability of human interferon-beta R27T variant, the method comprising a step of substituting serine for the 17th amino acid serine in the human interferon-beta R27T variant in which threonine is substituted for the 27th amino acid arginine of human interferon-beta.

## Description

### TECHNICAL FIELD

This application claims the priority of Korean Patent Application Nos. 10-2020-0052513 and 10-2020-0052913, filed on April 29, 2020, the entirety of which is a reference of the present application.

The present invention relates to a human interferon-beta variant with double mutation and a method for improving stability of a human interferon-beta variant and, more specifically, to a human interferon-beta variant comprising an amino acid sequence having serine substituted for the 17th amino acid cysteine of the human interferon-beta and threonine substituted for the 27th amino acid arginine of the human interferon-beta, and a method for improving stability of a human interferon-beta R27T variant, the method comprising a step of substituting serine for the 17th amino acid cysteine in the human interferon-beta R27T variant in which threonine is substituted for the 27th amino acid arginine of the human interferon-beta.

### BACKGROUND ART

Interferons (IFNs) are a type of cytokines, and have antiviral activity and have functions of inhibiting cell proliferation, and regulating natural immune responses, and among them, interferon-beta (IFN-β) is a globular protein with five alpha helices, in which the size is 22 kD, and becomes 18 kD when sugar chains are removed (Arduini etc., Protein Science 8: pp1867-1877, 1999).

Research on the clinical application of interferon-beta is being actively conducted, and in particular, the interferon-beta is in the spotlight as a drug for alleviating, reducing or treating symptoms for multiple sclerosis.

The interferon-beta has various immunological activities, such as antiviral activity, cell growth inhibition or antiproliferative activity, lymphocyte cytotoxicity enhancing activity, immunomodulatory activity, differentiation induction or inhibitory activity of target cells, activation activity of macrophages, increased activity of cytokine production, increased activity effect of cytotoxic T cells, increased activity of natural killing cells, etc., in addition to multiple sclerosis. Accordingly, it is reported that the interferon-beta is effective in treating cancer, autoimmune disorders, viral infections, HIV-related diseases, hepatitis C, rheumatoid arthritis, and the like.

Currently, there are two types of interferon-beta to be used for treatment. First, interferon-beta-1a is a glycosylated protein which is produced from the chinese hamster ovary (CHO) including a human interferon-beta gene, consists of 166 amino acid residues, and has a size of 22 kD. Second, interferon-beta-1b is a protein consisting of 165 amino acid residues produced from E. coil, in which a sugar is deleted, a methionine residue as the first amino acid is deleted, and serine is substituted for the 17th cysteine residue. Interferon-beta-1a which has been currently commercialized includes Rebif and Avonex, and interferon-beta-1b includes Betaseron and Extavia.

On the other hand, human interferon-beta is also a type of glycoprotein, and since the sugar chain portion linked to the protein plays an important role in the activity of the protein, in the case of the glycoprotein, the activity thereof may be increased when sugar chains are added. That is, it is known that the protein glycosylation may affect many biochemical properties, such as stability, solubility, intracellular trafficking activity, pharmacokinetics and antigenicity.

Accordingly, it has been reported an example of preparing a human interferon-beta variant with increased or improved activity or function by introducing a sugar chain into a glycoprotein, human natural interferon-beta (Korean Patent Registration No. 0781666). As used herein, a human interferon-beta variant, R27T is a recombinant human interferon-beta variant (hereinafter, rhINF-β) designed by substituting threonine (Thr) for arginine (Arg) at position 27 for additional glycosylation at position 25 of interferon-beta 1a, and exhibits effects of increased stability, decreased protein aggregation tendency, and increased half-life compared to wild-type interferon-beta 1a (Rebif). That is, R27T is a biobetter of rhINF-β generated by additional glycosylation through site-directed mutagenesis.

Meanwhile, one of the main tasks in the development of protein drugs is to provide a protein that provides sufficient chemical, physical, and biological stability to exhibit improved stability in a purification process and a storage process. However, it is still difficult to achieve high stability due to various intrinsic susceptibility in proteolytic pathways, and the complexity of protein structures with different levels of macromolecular, secondary, tertiary, and quaternary structures of the protein.

Insulin as a first recombinant peptide hormone was first approved in 1982 and has been successfully produced for more than 30 years, and currently, success cases of numerous recombinant protein/peptide drugs have been subsequently reported. However, in the development process of biopharmaceuticals, in particular, in formulation, there are still facing difficulties due to various factors such as protein aggregation, physicochemical instability, low half-life, low solubility, and pharmacokinetic properties.

In particular, protein aggregation and degradation are one of the major problems that occur easily in almost all biopharmaceutical processes, in which therapeutic proteins are structurally/thermodynamically instable in a solution during storage. Since the therapeutic proteins are sensitive to structural changes due to various factors during purification, processing, and storage, these problems may be exacerbated when the proteins are exposed to severe conditions such as repeated freeze/thawing and storage in buffers with different pH. In addition, since protein-based biopharmaceuticals have a possibility of physical degradation such as insoluble particle formation due to unfolding, aggregation, and non-native folding, it is important to avoid protein aggregation or physical denaturation and maximize stability.

In 2004, interferon-beta mutation proteins causing mutation to glycosylation of interferon have been developed (Korean Patent No. 781666), and studies to use the proteins as therapeutic agents have been conducted. Since the interferon-beta is less stable, in the purification process, degradation reactions, such as cleavage of peptide bonds, deamidation, and oxidation of methionine to methionine sulfide, and disulfide exchange, commonly occur (US2012/0177603), but it is necessary to consider these degradation reactions.

Accordingly, it is necessary to develop a human interferon-beta variant that exhibits more excellent efficacy than a pharmaceutical effect of natural interferon-beta, and a method for obtaining the human interferon-beta variant in high yield is required.

### DISCLOSURE

### TECHNICAL PROBLEM

Accordingly, the present inventors have made an effort to develop an interferon-beta variant with excellent pharmaceutical effect and improved purification efficiency compared to natural interferon-beta. As a result, the present inventors confirmed that a human interferon-beta variant comprising an amino acid sequence having serine substituted for the 17th amino acid cysteine and threonine substituted for the 27th amino acid arginine of the human interferon-beta had excellent interferon beta activity and excellent efficiency in a purification process, so as to be used for preparing new interferon beta.

In addition, the present inventors have conducted intensive research to develop a method for improving stability of an R27T variant, which was a human interferon-beta variant, more specifically, purification stability, storage stability, and freeze/thawing stability, found that it was possible to achieve the obj ect by substituting serine for the 17th amino acid cysteine, which was the 17th amino acid of the R27T variant, and then completed the present invention.

Therefore, an object of the present invention is to provide a human interferon-beta variant comprising an amino acid sequence having serine substituted for the 17th amino acid cysteine and threonine substituted for the 27th amino acid arginine of human interferon-beta.

Another object of the present invention is to provide a polynucleotide encoding the human interferon-beta variant.

Yet another object of the present invention is to provide an expression vector expressing human interferon-beta in an animal cell comprising the polynucleotide.

Yet another object of the present invention is to provide an animal cell transformed with the vector.

Yet another object of the present invention is to provide a method for preparing a human interferon-beta variant comprising culturing the animal cell.

Yet another object of the present invention is to provide a pharmaceutical composition comprising the human interferon-beta variant as an active ingredient.

Yet another object of the present invention is to provide a pharmaceutical composition consisting of the human interferon-beta variant as an active ingredient.

Yet another object of the present invention is to provide a pharmaceutical composition consisting essentially of the human interferon-beta variant as an active ingredient.

Yet another object of the present invention is to provide a method for improving stability of a human interferon-beta R27T variant, comprising substituting serine for the 17th amino acid cysteine in the human interferon-beta R27T variant in which threonine is substituted for the 27th amino acid arginine of human interferon-beta.

Yet another object of the present invention is to provide the use of the human interferon-beta variant for preparing an agent having a pharmaceutical effect of natural human interferon-beta on a disease selected from the group consisting of multiple sclerosis, cancer, autoimmune disorders, viral infection, HIV-related diseases, and hepatitis C.

Yet another object of the present invention is to provide a method for treating a disease selected from the group consisting of multiple sclerosis, cancer, autoimmune disorders, viral infection, HIV-related diseases, and hepatitis C, comprising administering an effective dose of a composition having a pharmaceutical effect of natural human interferon-beta and comprising the human interferon-beta variant to a subject in need thereof.

### TECHNICAL SOLUTION

In order to achieve the object, the present invention provides a human interferon-beta variant comprising an amino acid sequence having serine substituted for the 17th amino acid cysteine and threonine substituted for the 27th amino acid arginine of human interferon-beta.

In order to achieve another object, the present invention provides a polynucleotide encoding the human interferon-beta variant.

In order to achieve yet another object, the present invention provides an expression vector expressing human interferon-beta in an animal cell comprising the polynucleotide.

In order to achieve yet another object, the present invention provides an animal cell transformed with the vector.

In order to achieve yet another object, the present invention provides a method for preparing a human interferon-beta variant comprising culturing the animal cell.

In order to achieve yet another object, the present invention provides a pharmaceutical composition comprising the human interferon-beta variant as an active ingredient.

Further, the present invention provides a pharmaceutical composition consisting of the human interferon-beta variant as an active ingredient.

Further, the present invention provides a pharmaceutical composition consisting essentially of the human interferon-beta variant as an active ingredient.

In order to achieve yet another object, the present invention provides a method for improving stability of a human interferon-beta R27T variant, comprising substituting serine for the 17th amino acid cysteine in the human interferon-beta R27T variant in which threonine is substituted for the 27th amino acid arginine of human interferon-beta.

In order to achieve yet another object, the present invention provides the use of the human interferon-beta variant for preparing an agent having a pharmaceutical effect of natural human interferon-beta on a disease selected from the group consisting of multiple sclerosis, cancer, autoimmune disorders, viral infection, HIV-related diseases, and hepatitis C.

In order to achieve yet another object, the present invention provides a method for treating a disease selected from the group consisting of multiple sclerosis, cancer, autoimmune disorders, viral infection, HIV-related diseases, and hepatitis C, comprising administering an effective dose of a composition having a pharmaceutical effect of natural human interferon-beta and comprising the human interferon-beta variant to a subject in need thereof.

Hereinafter, the present invention will be described in detail.

The human interferon-beta variant with double mutation in the present invention is a human interferon-beta variant comprising an amino acid sequence having serine substituted for the 17th amino acid cysteine and threonine substituted for the 27th amino acid arginine of human interferon-beta.

In the present invention, the `human interferon-beta variant' represents all polypeptides having all or part of an amino acid sequence derived from human interferon-beta, and having the activity of the human interferon-beta while substituting serine for the 17th amino acid cysteine and threonine for the 27th amino acid arginine in the natural human interferon-beta.

The "activity of the human interferon-beta" used herein is defined as one or more activities that any polypeptide is sufficient to be identified as human interferon-beta among the known activities of the human interferon-beta. These activities may include, for example, reduction, alleviation or treatment activity for multiple sclerosis, antivirus activity, cell growth inhibition activity, anti-growth activity, anti-proliferative activity, lymphocyte cell toxicity enhancement activity, immune regulatory activity, differentiation induction or inhibition activity of target cells, increased activity of cytokine generation, increased effect activity of cytotoxic T cells, increased effect activity of macrophages, increased activity of natural killing cells, cancer prevention or treatment activity, automated immune disability prevention or treatment activity, virus infection prevention or treatment activity, prevention or treatment activity of HIV-related diseases, hepatitis C prevention or treatment activity, rheumatoid arthritis prevention or treatment activity, and the like.

The human interferon-beta variant with double mutation of the present invention is most preferably a polypeptide comprising an amino acid sequence having serine substituted for the 17th amino acid cysteine and threonine substituted for the 27th amino acid arginine in natural human interferon-beta having an amino acid sequence of SEQ ID NO: 1.

In the present invention, the variant having serine substituted for the 17th amino acid cysteine and threonine substituted for the 27th amino acid arginine in the natural human interferon-beta may comprise an amino acid sequence of SEQ ID NO: 3, specifically consist essentially of an amino acid sequence of SEQ ID NO: 3, more specifically consist of an amino acid sequence of SEQ ID NO: 3, but is not limited thereto.
SEQ ID NO: 1: 
MSYNLLGFLQRSSNFQCQKLLWQLNGRLEYCLKDRMNFDIPEEIKQLQ
QFQKEDAALTIYEMLQNIFAIFRQDSSSTGWNETIVENLLANVYHQINHLKTVLE
EKLEKEDFTRGKLMSSLHLKRYYGRILHYLKAKEYSHCAWTIVRVEILRNFYFI
NRLTGYLRN
SEQ ID NO: 3:

MSYNLLGFLQRSSNFQSQKLLWQLNGTLEYCLKDRMNFDIPEEIKQLQ

QFQKEDAALTIYEMLQNIFAIFRQDSSSTGWNETIVENLLANVYHQINHLKTVLE
EKLEKEDFTRGKLMSSLHLKRYYGRILHYLKAKEYSHCAWTIVRVEILRNFYFI
NRLTGYLRN

In addition, the human interferon-beta variant of the present invention may be a human interferon-beta variant which has serine substituted for the 17th amino acid cysteine and threonine substituted for the 27th amino acid arginine of the human interferon-beta of SEQ ID NO: 1, has at least 90% sequence homology with wild-type interferon beta of SEQ ID NO: 1, and has the activity of interferon-beta.

The term "variant" as used herein refers to a protein in which one or more amino acids are different from the recited sequence in conservative substitutions and/or modifications, but functions or properties of the protein are maintained. The variant is different from an identified sequence by substitution, deletion, or addition of several amino acids. Such a variant may generally be identified by modifying one or more amino acids of the amino acid sequence or the protein and evaluating the modified protein. That is, the ability of the variant may be increased, unchanged, or decreased compared to a native protein. In addition, some variants may include variants in which one or more portions, such as an N-terminus leader sequence or a transmembrane domain, have been removed. Other variants may include variants in which a portion thereof is removed from an N- and/or C-terminus of a mature protein. The term "variant" may be used with modification, modified protein, modified polypeptide, mutant, mutein, divergent, variant, etc. (in English expressions), and any term used in a modified meaning is not limited thereto. For the purposes of the present invention, the variant may have increased activity of the mutated protein compared to a natural wild-type or unmodified protein, but is not limited thereto.

The term "conservative substitution" used herein means substituting one amino acid with another amino acid having a similar structural and/or chemical property. The variant may have, for example, one or more conservative substitutions while still having one or more biological activities. These amino acid substitutions may generally occur based on polarity, charge, solubility, and similarity in hydrophobic, hydrophobic and/or amphipathic nature of residuals. For example, among amino acids with electrically charged amino acid pendants, positively charged (basic) amino acids include arginine, lysine, and histidine, and negatively charged (acidic) amino acids include glutamic acid and aspartic acid. Among amino acids with uncharged amino acid pendants, nonpolar amino acids include glycine, alanine, valine, leucine, isoleucine, methionine, phenylalanine, tryptophan and proline, and polar or hydrophilic amino acids include serine, threonine, cysteine, tyrosine, asparagine and glutamine. Among the nonpolar amino acids, aromatic amino acids include phenylalanine, tryptophan and tyrosine.

In addition, the variant may further include deletion or addition of amino acids that have a minimal effect on the properties and secondary structure of the polypeptide. For example, the polypeptide may be conjugated with a signal (or leader) sequence at the N-terminus of the protein involved in the transfer of the protein co-translationally or post-translationally.

In one aspect of the present invention, the human interferon-beta variant may include an amino acid sequence having threonine fixed for the 27th amino acid and serine fixed for the 17th amino acid of the wild-type human interferon-beta protein of SEQ ID NO: 1 and having at least 80% homology or identity, but is not limited thereto. Specifically, the variant of the present invention may include a protein having at least 80%, 85%, 90%, 95%, 96%, 97%, 98%, or 99% homology or identity with an amino acid sequence of SEQ ID NO: 3. In addition, if the variant is an amino acid sequence having such homology or identity and exhibiting efficacy corresponding to the protein, in addition to the amino acids at positions 27 and 17 of the wild-type human interferon-beta protein of SEQ ID NO: 1, it is obvious that a protein having an amino acid sequence in which some of the sequence are deleted, modified, substituted or added is also included within the scope of the present disclosure.

The term "homology" or "identity" used herein means a degree associated with two given amino acid sequences and may be represented as a percentage. The term 'homology' or 'identity' may be often used interchangeably.

Whether any two protein sequences have homology, similarity or identity may be determined using known computer algorithms, such as a "FASTA" program using default parameters as known in the art. Alternatively, a Needleman-Wunsch algorithm, as performed in the Needleman program (version 5.0.0 or later) of an EMBOSS package, may be used and determined (including a GCG program package). For example, the homology, similarity, or identity may be determined using BLAST, or ClustalW at the National Center for Biotech Information Database.

In addition, the present invention provides a polynucleotide encoding a human interferon-beta variant with double mutation of the present invention.

As used herein, the term "polynucleotide" is defined as a meaning that includes either single-stranded or double-stranded RNA, DNA, or RNA-DNA polymers.

Those skilled in the art may easily prepare a polynucleotide encoding the amino acid sequence based on the given amino acid sequence so long as using his or her ordinary abilities.

The present invention also provides an animal cell expression vector comprising the polynucleotide capable of expressing the human interferon-beta variant of the present invention in an animal cell.

The human interferon-beta variant with double mutation of the present invention further includes 1 or 2 sugar chains compared to natural human interferon-beta variant, but considering that these sugar chains generally occur in the animal cell, the animal cell expression vector specifically basically includes:
(i) a polynucleotide encoding the human interferon-beta variant as described above;
(ii) a promoter operably linked to the nucleotide sequence of (i) to form an RNA molecule;
(iii) a polynucleotide encoding a leader sequence;
(iv) an origin of replication; and
(v) a 3'-non-translational site that causes polyadenylation of a 3'-terminus of the RNA molecule.

In the above, the promoter refers to a sequence capable of activating transcription, but such a sequence is known in the art, and similarly, the 3'-non-translational site, which serves to stabilize the leader sequence and mRNA for transport of the translated protein to the endoplasmic reticulum where glycosylation occurs, is also known in the art.

Meanwhile, the expression vector of the present invention may include optionally a reporter (e.g., luciferase and β-glucuronidase) gene, an antibiotic (e.g., neomycin, carbenicillin, kanamycin, spectinomycin, hygromycin, etc.) resistance gene a selection marker gene, or the like, and may optionally include an enhancer.

Meanwhile, examples that may be used as the animal cell expression vector of the present invention may include pSV2-neo, pCAGGS, pcDL-SRa296, pAc373, and the like, but the exemplified vectors may include the promoter, the leader, the 3'-non-translational site, the reporter gene, the selection marker gene, the enhancer, and the like as described above if necessary.

The present invention provides an animal cell transformed with the expression vector and a method for producing a human interferon-beta variant by culturing the animal cell.

As used herein, the transformation refers to modification of a genotype of a host cell by introducing an exogenous polynucleotide (in the present invention, meaning a polynucleotide encoding the human interferon-beta variant with double mutation) and means introducing an exogenous polynucleotide into the host cell regardless of a method used for the transformation. The exogenous polynucleotide introduced into the host cell may be integrated and maintained or not integrated but maintained into a genome of the host cell, and the present invention includes both thereof.

Meanwhile, as used herein, the animal cell includes a mammalian cell and an insect cell that may be used for the production of recombinant proteins. Examples of the animal cell that may be used in the present invention may include COS cells, CHO cells, C-127 cells, BHK cells, rat Hep I cells, rat Hep II cells, TCMK cells, human lung cells, human liver tumor cells, HepG2 cells, mouse hepatocytes, DUKX cells, 293 cells, and the like, and examples of the insect cell may include silkworm culture cells.

In yet another aspect, the present invention relates to a pharmaceutical composition comprising the human interferon-beta variant of the present invention as described above.

The human interferon-beta included in the pharmaceutical composition of the present invention has been mainly used as a therapeutic agent for multiple sclerosis, but it is reported to be used for the treatment of cancer, autoimmune disorders, viral infections, HIV-related diseases, hepatitis C, etc., and a pharmaceutical effect has been continuously reported.

For this reason, it should be understood that the pharmaceutical effect of the pharmaceutical composition of the present invention includes not only a pharmaceutical effect as a therapeutic agent for multiple sclerosis, but also all other pharmaceutical effects of human interferon-beta.

In addition, such a pharmaceutical effect should be understood as a meaning that includes not only pharmaceutical effects known to date, but also pharmaceutical effects to be found later as the pharmaceutical effect of human interferon-beta.

Since the pharmaceutical composition of the present invention is characterized by including a human interferon-beta variant with increased activity or function obtained by the present invention, even if the pharmaceutical composition of the present invention includes not only pharmaceutical effects known to date but also pharmaceutical effects to be found later of the drug, the scope of the present invention is not unreasonably expanded.

Nevertheless, the human interferon-beta variant has been mainly used as a therapeutic agent for multiple sclerosis, and the therapeutic effects of cancer, autoimmune disorders, viral infections, HIV-related diseases, hepatitis C, etc. have already been found, so that the pharmaceutical effect is preferably these pharmaceutical effects.

Meanwhile, the pharmaceutical composition of the present invention may be administered orally or through other routes comprising transdermal, subcutaneous, intravenous or intramuscular.

Alternatively, the pharmaceutical compositions of the present invention may be prepared in various formulations, and in the case of formulation, may be prepared using commonly used diluents or excipients such as fillers, extenders, binders, wetting agents, disintegrants and surfactants.

In addition, in the daily dose of the pharmaceutical compositions of the present invention, the pharmaceutical compositions may be administered at a amountin already known in the art, but may be generally administered once or divided into several times in the body weight range of 0.01 to 5 mg/kg. However, since the actual dose of the pharmaceutical composition of the present invention is determined according to several related factors such as a route of administration, the age, sex and body weight of a patient, and the severity of the patient, it should not be understood that the dose limits the scope of the present invention in any aspect.

The present invention also provides a method for improving stability of a human interferon-beta R27T variant, comprising substituting serine for the 17th amino acid cysteine in the human interferon-beta R27T variant in which threonine is substituted for the 27th amino acid arginine of human interferon-beta.

In an embodiment of the present invention, after serine is substituted and purified for the 17th amino acid cysteine in the human interferon-beta R27T variant (C17S), changes in glycosylation of the variants with and without the C17S mutation were confirmed by using RP-HPLC. As a result, it was confirmed that the 2 glycosylation ratio of the R27T variant with C17S after protein purification was improved compared to the R27T variant without C17S.

In another embodiment of the present invention, after serine is substituted and purified for the 17th amino acid cysteine in the human interferon-beta R27T variant (C17S), storage stability in a phosphate buffer and an acetic acid buffer of pH 2.0 to 6.0 was evaluated. As a result, it was confirmed that the protein recovery rate in each buffer of the R27T variant with C17S was improved compared to the R27T variant without C17S.

In another embodiment of the present invention, after serine is substituted and purified for the 17th amino acid cysteine of the human interferon-beta R27T variant (C17S), SEC-HPLC analysis was performed while repeating freeze/thawing to confirm the ratio of protein monomers. As a result, it was confirmed that after the repeated freeze/thawing process, the monomer ratio of the R27T variant with C17S was improved compared to the R27T variant without C17S.

In the present invention, the "human interferon-beta R27T variant" represents all polypeptides that have all or part of the amino acid sequence derived from the human interferon-beta and have the activity of human interferon-beta while substituting threonine for the 27th amino acid arginine in wild-type human interferon-beta of SEQ ID NO: 1.

In the present invention, the human interferon-beta R27T variant having threonine substituted for the 27th amino acid arginine of the human interferon-beta may comprise an amino acid sequence of SEQ ID NO: 2, specifically consist essentially of an amino acid sequence of SEQ ID NO: 2, more specifically consist of an amino acid sequence of SEQ ID NO: 2, but is not limited thereto.
SEQ ID NO: 2:

MSYNLLGFLQRSSNFQCQKLLWQLNGTLEYCLKDRMNFDIPEEIKQLQ
QFQKEDAALTIYEMLQNIFAIFRQDSSSTGWNETIVENLLANVYHQINHLKTVLE
EKLEKEDFTRGKLMSSLHLKRYYGRILHYLKAKEYSHCAWTIVRVEILRNFYFI
NRLTGYLRN

In addition, the human interferon-beta R27T variant may include an amino acid sequence having threonine fixed for the 27th amino acid of the wild-type human interferon-beta protein of SEQ ID NO: 1 and having at least 80% homology or identity thereof, but is not limited thereto. Specifically, the variant of the present invention may include a protein having at least 80%, 85%, 90%, 95%, 96%, 97%, 98%, or 99% homology or identity with an amino acid sequence of SEQ ID NO: 2. In addition, if the variant is an amino acid sequence having such homology or identity and exhibiting efficacy corresponding to the protein, in addition to the amino acid at position 27 of the wild-type human interferon-beta protein of SEQ ID NO: 1, it is obvious that a protein having an amino acid sequence in which some of the sequence are deleted, modified, substituted or added is also included within the scope of the present disclosure.

In the present invention, the step of substituting serine for the 17th amino acid cysteine of the human interferon-beta R27T variant may be performed by known methods used to introduce point mutations of amino acids in the art without limitation.

For example, the step may be performed by a method of transforming a host cell with a vector including a polynucleotide coding the protein having threonine substituted for the 27th amino acid arginine and serine substituted for the 17th amino acid cysteine of the wild-type human interferon-beta protein of SEQ ID NO: 1 and then culturing the host cell in a medium.

For a detailed description related thereto, the contents of the method for producing the human interferon-beta variant comprising R27T and C17S double mutation may be applied as it is.

In the present invention, the polynucleotide coding the human interferon-beta variant may include any polynucleotide sequence coding the protein having threonine substituted for the 27th amino acid arginine and serine substituted for the 17th amino acid cysteine of the wild-type human interferon-beta of SEQ ID NO: 1 without limitation. Specifically, in the present invention, the polynucleotide may be variously modified in coding regions within a range without changing the amino acid sequence of the protein due to codon degeneracy or in consideration of a codon preferred in an organism to express the protein.

The human interferon-beta variant produced by the culture may be released into the medium or may not be released into the medium but remain in the cell.

In the method of the present invention, a step of recovering the human interferon-beta R27T variant having serine substituted for the 17th amino acid cysteine from the cultured cell or medium may be further included.

The method for recovering the human interferon-beta variant produced in the culturing step may be collecting a target protein from the culture medium using a suitable method known in the art according to the culture method. For example, centrifugation, filtration, anion exchange chromatography, crystallization, HPLC, and the like may be used, and it is possible to recover desired variants from the medium or cell by using a proper method known in the art.

In addition, the recovering step may include a purification process, and may be performed using a suitable method known in the art, for example, filtration using a membrane and the like.

In an aspect of the present invention, the stability may be selected from the group consisting of purification stability, storage stability, and freeze/thawing stability.

The purification stability means that the rate of protein denaturation that may occur in the process of purifying the protein recovered from the host cell or cell culture medium is lowered, and the purification may include, without limitation, conventional methods for purifying the protein in the art. For example, the purification includes salting out (e.g., ammonium sulfate precipitation, and sodium phosphate precipitation), solvent precipitation (precipitation of protein fraction using acetone, ethanol, etc.), dialysis, gel filtration, ion exchange, and column chromatography such as reverse phase column chromatography, and may be preferably column chromatography.

In one aspect of the present invention, the purification stability may mean that a change in glycosylation level of the protein is lowered before and after the purification process of the protein described above, and specifically, may mean that the purification rate of the 2 glycosylated protein is improved in the human interferon-beta R27T variant.

In another aspect of the present invention, the purification stability may be characterized in that protein aggregation and degradation are reduced during concentration and buffer exchange of the protein. In general, the proteins or polypeptides may be aggregated or degraded in the concentration and buffer exchange process performed during/or after the purification process, but when serine is substituted for the 17th amino acid cysteine of the human interferon-beta R27T variant, the protein denaturation in the concentration and buffer exchange process may be reduced.

The proteins may be concentrated using methods known in the art. Non-limiting exemplary methods that may be used for protein concentration include ultrafiltration, tangential flow filtration, centrifugal concentration using a membrane concentrator (e.g., a cellulose membrane concentrator), dialysis against a water absorbing material (e.g., a water absorbing polymer), salting out (e.g., using ammonium sulfate), and chromatography (e.g., size exclusion chromatography).

The ultrafiltration for protein concentration is an isolation method using water pressure so that molecules and solvents pass through a membrane made of pores known as a particle size and also a cut-off size of the value. Since molecules with a higher molecular weight do not pass through the membrane, only molecules with a molecular weight smaller than the cut-off value of the membrane may pass through the membrane and form a so-called preservation solution. Accordingly, the molecules present in the preservation solution are concentrated as the solvent flows across the membrane. The ultrafiltration may be used for protein concentration or buffer exchange, or may be used to formulate a target protein into a desired solution or a desired buffer.

In a specific embodiment, the concentration of a solution or composition comprising a target protein may be performed by tangential flow filtration (TFF). This method is particularly useful for concentration on large scales, i.e., for concentration of the solution in a volume from 1 liter to several hundred liters. Therefore, this method is particularly useful for the production of concentrated solutions of the target protein on an industrial scale.

The TFF technique is based on the use of a specific device that allows the filtered solution to flow across a semi-permeable membrane through which only molecules smaller than the pores of the membrane may pass, forms a filtrate, and leaves a larger material to be collected (holding amount). Two different pressures are applied with the TFF method; one pressure is to feed a solution into a system to circulate the solution within the system (inlet pressure), and the other pressure is applied across a membrane causing small molecules and solvents to cross the membrane (membrane pressure). The inlet pressure may typically be in the range of 1 to 3 bar, for example, 1.5 to 2 bar. The membrane pressure may typically be greater than 1 bar.

When the TFF is used to concentrate the composition, the concentrated composition of the target protein may be collected a buffer. A membrane useful for TFF may typically be made of regenerated cellulose or polyethersulfone (PES). The pore size of the membrane may typically have a molecular weight cutoff of less than 10,000 Mw, for example, in the range of 10 to 10,000 Mw.

In another embodiment, the concentration of the composition comprising the target polypeptide may be performed by use of a centrifugation device. In this case, the target protein is filtered by the membrane by application of centrifugal force to the membrane. Such a membrane is often characterized by molecular weight (Mw) cutoff, i.e., a maximum molecular size of a compound that may pass through the membrane, and compounds with a larger molecular size therethan may not pass through the membrane.

The membrane may be made of particularly polyethersulfone (PES) or regenerated cellulose. An example of such a suitable commercial filter device may be Centricon Plus-80 or Centricon Plus-15, but is not limited thereto.

The concentration may generally be performed at 2000 to 4500 g, for example, 2500 to 4000 g, or 2750 to 3500 g, or 3000 to 3500 g, for example, 3000 g or 3100 g or 3200 g or 3300 g or 3400 g or 3500 g.

The buffer exchange of the composition comprising the concentrated target protein may be performed by a) diluting, for example, 5 to 15 times the composition comprising the target protein concentrated in a buffer or formulation, and b) concentrating the diluted composition again and performing the step, and then configuring additives of the buffer or formulation in the composition so that the amount the additives of the buffer or formulation in the composition before these steps is, for example, 5 v/v% or less or 1 v/v% or less.

In the present invention, the storage stability means that the protein denaturation rate that may occur in a process of storing the purified human interferon-beta R27T variant in a buffer or changing the composition of the buffer is lowered.

In the present invention, the buffer may have pH 2.0 to 6.0, preferably pH 2.0 to 5.0, and most preferably pH 2.0 to 4.0, but is not limited thereto.

In the present invention, the buffer may be selected from the group consisting of acetic acid, phosphoric acid, ammonium carbonate, ammonium phosphate, boric acid, citric acid, lactic acid, potassium citrate, potassium metaphosphate, potassium phosphate monobasic, sodium acetate, sodium citrate, sodium lactate solution, dibasic sodium phosphate, monobasic sodium phosphate, bicarbonate, tris(tris(hydroxymethyl)aminomethane), 3-(N-morpholino)propanesulfonic acid (MOPS), N-(2-hydroxyethyl)piperazine-N'-(2-ethanesulfonic acid) (HEPES), 2-(2-amino-2-oxoethyl)aminoethanesulfonic acid (ACES), N-(2-acetamido)2-iminodiacetic acid (ADA), 3-(1,1-dimethyl-1,2-hydroxyethylamino-2-propanesulfonic acid (AMPSO), N,N-bis(2-hydroxyethyl)-2-aminoethanesulfonic acid (BES), N,N-bis(2-hydroxyethylglycine (Bicine), bis-tris (bis-(2-hydroxyethyl)imino-tris(hydroxymethyl)methane, 3-(cyclohexylamino)-1-propanesulfonic acid (CAPS), 3-(cyclohexylamino)-2-hydroxy-1-propanesulfonic acid (CAPSO), 2-(N-cyclohexylamino)ethanesulfonic acid (CHES), 3-N,N-bis(2-hydroxyethylamino-2-hydroxy-propanesulfonic acid (DIPSO), N-(2-hydroxyethylpiperazine)-N'-(3-propanesulfonic acid) (HEPPS), N-(2-hydroxyethyl)piperazine-N'-(2-hydroxypropanesulfonic acid (HEPPSO), 2-(N-morpholino)ethanesulfonic acid (MES), triethanolamine, imidazole, glycine, ethanolamine, phosphate, 3-(N-morpholino)-2-hydroxypropanesulfonic acid (MOPSO), piperazine-N,N'-bis(2-ethanesulfonic acid (PIPES), piperazine-N,N'-bis(2-hydroxypropanesulfonic acid (POPSO), N-trishydroxymethyl)methyl-3-aminopropanesulfonic acid (TAPS), 3-N-tris(hydroxymethyl)methylamino-2-hydro hydroxy-propanesulfonic acid (TAPSO), N-tris(hydroxymethyl)methyl-2-aminoethanesulfonic acid (TES), N-tris(hydroxymethyl)methylglycine (Tricine), 2-amino-2-methyl-1,3-propanediol, and 2-amino-2-methyl-1-propanol, but is not limited thereto.

In the present invention, the freeze/thawing stability means that when a cycle of freezing and thawing the human interferon-beta R27T variant stored in the buffer is repeated, a possibility of protein denaturation is lowered.

In one aspect of the present invention, the freeze/thawing stability may be characterized as thawing stability after freezing at - 100°C to - 10°C, preferably thawing stability after freezing at - 90°C to - 30°C, and most preferably thawing stability after freezing at - 80°C to - 50°C.

In another aspect of the present invention, the freeze/thawing stability may be characterized as freeze/thawing stability in an acetate buffer, preferably freeze/thawing stability in an acetate buffer of pH 3.0 to 5.0.

In another aspect of the present invention, the freeze/thawing stability may be characterized as reduced aggregation and degradation of proteins after three or more freeze/thawing cycles, preferably reduced aggregation and degradation of proteins after four or more freeze/thawing cycles, and more preferably reduced aggregation and degradation of proteins after four or more freeze/thawing cycles.

The biological activity of the human interferon-beta may be changed by interaction with a Type 1 interferon receptor. In the present invention, in order to improve the stability of the human interferon-beta R27T variant, the mutation-induced 17th amino acid is located on a binding surface with IFNAR2, a type 1 interferon receptor, and particularly, the 15th to 23rd sites are major receptor binding sites, so that the biological activity may be also changed when any one or more of the amino acid sequence are mutated. In particular, since a free cysteine residue has stronger hydrophobicity than disulfide bonding cysteine, the change in the activity may be caused by substituting the 17th amino acid cysteine. As expected, due to the introduction of an additional C17S mutation to the human interferon-beta R27T variant, a change in the hydrophobicity of the R27T variant was lowered, but there was no effect on protein activity, and the purification stability, storage stability, and freeze/thawing stability were improved. In addition, the introduction of the C17S mutation may improve stability by inducing hydrogen bonding in the protein by serine residues.

The present invention provides the use of the human interferon-beta variant for preparing an agent having a pharmaceutical effect of natural human interferon-beta on a disease selected from the group consisting of multiple sclerosis, cancer, autoimmune disorders, viral infection, HIV-related diseases, and hepatitis C.

The present invention provides a method for treating a disease selected from the group consisting of multiple sclerosis, cancer, autoimmune disorders, viral infection, HIV-related diseases, and hepatitis C, comprising administering an effective dose of a composition having a pharmaceutical effect of natural human interferon-beta and comprising the human interferon-beta variant to a subject in need thereof.

The term `effective dose' of the present invention means an amount that exhibits effects of improving, treating, preventing, detecting, and diagnosing of a disease selected from the group consisting of multiple sclerosis, cancer, autoimmune disorders, viral infection, HIV-related diseases, and hepatitis C, or inhibiting or alleviating the disease when administered to the subject. The 'subject' may be animals, preferably, mammals, particularly animals comprising humans, and may also be cells, tissues, organs, and the like derived from animals. The subject may be a patient requiring the effects.

The term 'treatment' of the present invention comprehensively refers to improving a disease selected from the group consisting of multiple sclerosis, cancer, autoimmune disorders, viral infection, HIV-related diseases, and hepatitis C or symptoms of the disease. The treatment may include treating or substantially preventing the disease, or improving the condition thereof and include alleviating, treating or preventing a symptom or most of symptoms derived from the cancer, but is not limited thereto.

The term 'comprising' as used herein is used in the same meaning as 'including' or `characterized by', and does not exclude additional ingredients or steps of the method which are not specifically mentioned in the composition or the method according to the present invention. The term `consisting of' means excluding additional elements, steps or ingredients, etc., unless otherwise described. The term `consisting essentially of' means including materials or steps which do not substantially affect basic properties thereof in addition to the described materials or steps within the range of the composition or the method.

### ADVANTAGEOUS EFFECTS

Therefore, the human interferon-beta variant with double mutation provided by the present invention greatly improves the efficiency in the purification process while having excellent the interferon-beta activity, so as to be used in the production of a therapeutic agent using the same.

In addition, according to the method for improving the stability of the human interferon-beta R27T variant of the present invention, it is possible to uniformly secure the protein quality in the preparing and distribution process by improving the protein stability in the purification process, the storage process and the freeze/thawing process while maintaining the activity of the human interferon-beta R27T variant having threonine substituted for the 27th amino acid arginine of the human interferon-beta.

### DESCRIPTION OF DRAWINGS

FIG. 1 illustrates PCR conditions in a protein expression DNA production experiment.
FIG. 2 illustrates restriction enzyme treatment and cloning in the protein expression DNA production experiment.
FIG. 3 illustrates the progress of cloning using T4 DNA ligase (NEB) in the protein expression DNA production experiment.
FIG. 4 illustrates colony PCR conditions in a protein expression DNA production experiment.
FIGS. 5A and 5B illustrate observation results of cell viability after transduction of ABN 101 (NT) and ABN 101 (CS) (ABN 101 (NT): R27T mutant interferon-beta, ABN 101 (CS): C17S, R27T double mutation interferon-beta).
FIGS. 6A and 6B illustrate 50 ml scale Fed-batch results of ABN 101 (NT) and ABN 101 (CS).
FIG. 7 illustrates 1 L scale Fed-batch results of ABN 101 (NT) and ABN 101 (CS).
FIG. 8 illustrates concentration and buffer exchange in an interferon-beta variant stability confirmation experiment.
FIGS. 9A and 9B illustrate interferon-variant RP-HPLC results.
FIGS. 10A to 10D illustrate changes in monomer content during interferon-variant buffer exchange.
FIGS. 11A to 11F illustrate confirmation of F/T stability comparison of interferon-variants in a 20 mM Na-Pi buffer.
FIGS. 12A to 12C illustrate confirmation of F/T stability comparison of interferon-variants in a 20 mM Na-OAc buffer.

### MODES FOR THE INVENTION

Hereinafter, the present invention will be described in detail.

However, the following Examples are just illustrative of the present invention, and the contents of the present invention are not limited to the following Examples.

The following experiments were performed to prepare ABN 101 (NT), R27T mutation human interferon beta-1a, and ABN 101 (CS), a R27T and C17S double mutation form.

### Experiment method

### 1. Preparation of protein expression DNA

To clone ABN 101 (NT) and ABN 101 (CS) behind a human promoter of a pD2535nt-HDP vector, primers added with XbaI and PacI enzyme restriction sites were designed, and sequences were as follows.
Forward: 5'-ggtctagagccaccAtgacca-3' (SEQ ID NO. 4)
   XbaI
Reverse: 5'-cacttagggattaattaatcagttcctcaggtag-3' (SEQ ID NO. 5)
   PacI

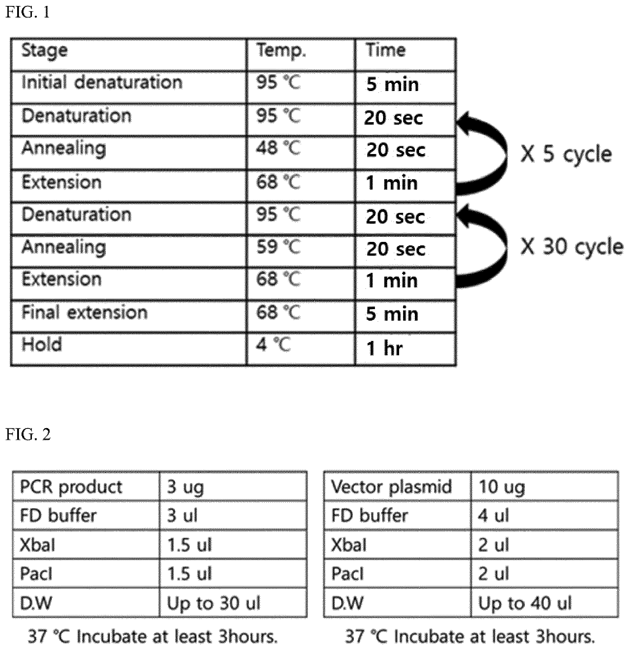

Since two inserts changed cysteine to serine by changing only the 119th DNA sequence, the primers for cloning were used in common. The two inserts were amplified through AccuPower PCR PreMix (Bioneer), and PCR conditions were shown in FIG. 1 below.

The size of a PCR product obtained through PCR was confirmed through 0.8% agarose gel, and gel extraction was performed using a MEGAquick-spin^{™} Plus Total Fragment DNAPurification Kit (Intron). The PCR product and the pD2535nt-HDP vector that had been purified were treated with restriction enzymes as illustrated in FIG. 2 to perform cloning. The restriction enzymes and the buffers thereof were all used with ThermoFisher products.

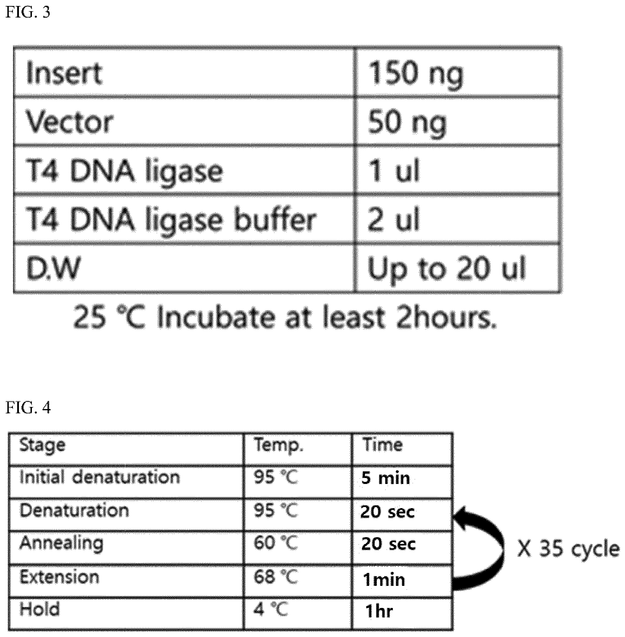

After restriction enzyme treatment, it was attempted to increase cloning efficiency by obtaining only pure DNA fragments using the MEGAquick-spin^{™} Plus Total Fragment DNA Purification Kit (Intron), and after purification, T4 DNA ligase (NEB) was used as illustrated in FIG. 3 to perform cloning.

After ligation, transformation was performed using a DH5a Chemically Competent E. coli (Enzynomics) product. First, DH5a cells were slowly thawed in ice, and then 20 µl of a reaction product was fully added and placed on ice for 30 minutes. Then, heat shock was applied at 42°C for 30 seconds and then stabilized on ice for 2 minutes. 400 µl of SOC media (provided by enzymenomics) were added and shaking-incubated at 37°C for 1 hour. After healing, cells centrifuged at 3000 rpm for 3 minutes were smeared in an LB medium containing 50 µg kanamycin, and then incubated overnight in a 37°C incubator. Colonies appearing on a plate were detached and colony PCR was performed to confirm cloning. A single colony without overlapping with other colonies was scraped as much as possible using a 10p tip and then buried in an AccuPower PCR PreMix (Bioneer) tube. PCR was performed with primers for colony PCR of a pD253 5nt-HDP vector provided by Horizon, and the conditions were as illustrated in FIG. 4 below.

The size of the PCR product was confirmed through 0.8% agarose gel to confirm the completion of cloning, and in the cloned pD2535nt-HDP::ABN101 (NT) and pD2535nt-HDP: :ABN101(CS), DNA prep for CHO-K1 cell transfection was performed through nucleobond Xtra Maxi Plus (MACHEREY-NAGEL).

### 2. Transduction

E. coli having an expression plasmid was incubated and harvested in the LB medium containing 100 µg/mL kanamycin, and DNA was isolated using a QIAGEN Plasmid Midi prep kit. 30 µl of a 10x cutsmart buffer and 2.5 µl of an NrU1-HF restriction enzyme were added to 50 µg of the isolated DNA to make a final volume of 300 µl. Then, the isolated DNA was incubated at 37°C for 2 hours to be linearized. After 2 hours, 30 µl of 1/10 volume of 3 M, pH 5.5 sodium acetate solution and 750 µl of ice-cold ethanol were added and incubated overnight at - 80°C. The next day, ethanol-precipitated DNA was centrifuged and washed with 70% ethanol to obtain high-purity DNA, and the concentration was measured using Nanodrop. On the first day for transduction, CHO-K1 cells were seeded in an E125 shake flask to be 3 x 10⁵ /ml using a CDFortiCHO culture medium added with L-Glutamine at a concentration of 4 mM and then incubated for 24 hours at 37°C, 5% CO₂, and 125 rpm conditions. On the second day, the number of cells seeded on the first day was measured and the cells were seeded to be 5 x 10⁵ /ml and incubated for 24 hours at 37°C, 5% CO₂, and 125 rpm conditions. On the third day, the number of seeded cells was measured to confirm whether the number has reached 1 × 10⁶ /ml, and when reached, the cells were finally seeded to be 1 × 10⁶ /ml to prepare the transduction. 37.5 µg of linearized DNA and 37.5 µl of a Freestyle MAX reagent were added to 600 µl of an OptiPRO SFM medium, and reacted at room temperature for 5 minutes. Thereafter, the mixture containing DNA was transferred to the mixture containing the Freestyle MAX reagent and mixed, and then reacted at room temperature for 25 minutes. After the reaction, the DNA-lipid mixture was carefully added to the previously seeded CHO-K1 cells. The cells were incubated for 48 hours at 37°C, 5% CO₂, and 125 rpm conditions to perform the transduction. After 48 hours of transduction, the selection of methionine sulfoximine (MSX) resistant cells was started. While the cells were incubated for about 25 days in a selective medium added with 25 or 50 µM of MSX, the fully transfected cells were cultured and monitored once every 2 to 3 days. While monitoring every 2 to 3 days, when the viability reached 70% or more and the number of viable cells reached 0.5 to 1.2 × 10⁶ /ml, these cells were selected as a pool, and a suspension culture was 3 or more subcultured on a selective medium at 37°C, 5% CO₂, and 125 rpm conditions to stabilize cells.

### 3. Fed-batch

Fed-batch was performed using the cells in the pool state prepared by transduction. 50 ml of the cells were seeded into an E250 shake flask to be 3 × 10⁵ /ml using a CDFortiCHO medium without MSX, and incubated for about 12 days at 37°C, 5% CO₂, and 125 rpm conditions. At this time, the glucose metabolites of the cell culture medium were analyzed using cedex bio, and the viability of cells and the number of viable cells were measured. On days 3, 5, and 7, a 5% (V/V) CD Efficient Feed C+ solution was added, and on days 4 and 6, a 45% glucose solution was added. After the Fed-batch was completed, the culture medium was centrifuged, and only a supernatant was taken and stored under a refrigerated or frozen condition.

### 4. Confirmation of interferon beta-1a biological activity (ELISA)

How much biological activity of the protein expressed by human Interferon beta-1a was exhibited with the prepared expression cell line was analyzed using a HuIFN-β ELISA KIT of TORAY Co., Ltd. The culture medium secured by Fed-batch was initially diluted 10,000-fold using a diluent in the KIT, and serially diluted two-fold to prepare a 1,280,000-fold diluted sample. A standard material for measuring the biological activity in the KIT was prepared using the diluent from 200 IU/ml to 3.125 IU/ml according to the protocol. An ELISA plate was primed and prepared using a wash buffer in the KIT. After priming, 100 µl/well of the prepared sample and the standard material were added to the ELISA plate, and 50 µl/well of a HRP-conjugated antibody was added to each well. The plate was incubated at 27°C for 2 hours and reacted. After the reaction was completed, the plate was washed, and then added with a color development reagent at 100 µl/well and incubated at 27°C for 30 minutes to induce a color development reaction. Thereafter, in order to complete the color reaction, a stop solution was added at 100 µl/well, and plate detection was performed at a wavelength of measurement 450 nm/reference 620 nm using a spectrophotometer device. Based on the absorbance obtained herein, a standard curve was drawn using a 4-parameter method and the biological activity of the sample was converted. The biological activity of the original sample was checked again by multiplying the activity of the sample obtained by conversion from the standard curve by a dilution rate.

### 5. Confirmation of expression (concentration) of interferon beta-1a (ELISA)

How much the protein expressed by human Interferon beta-1a was expressed with the prepared expression cell line was analyzed using a HuIFN-β ELISA kit of TORAY Co., Ltd. In the same manner as the biological activity confirmation method, the culture medium secured by Fed-batch was initially diluted 10,000-fold using a diluent in the KIT, and serially diluted two-fold to prepare a 1,280,000-fold diluted sample. The existing reference standard material was prepared by two-fold serial dilution at a concentration from 2.5 ng/ml to 0.039 ng/ml. The subsequent ELISA test process was the same as the biological activity confirmation ELISA test method. Based on the obtained absorbance, a standard curve was drawn using a 4-parameter method and the Interferon beta-1a expression level of the sample was converted. The concentration of the original sample was confirmed again by multiplying the expression level obtained by conversion from the standard curve by the dilution rate.

### 6. Purification of interferon-beta variant

The cell line prepared in Example above was incubated using a cell factory (Nunc Co., Ltd., Cat No. 170069). Each expression cell line was sub-cultured into the cell factory at 5 × 10⁴ cells/ml with an alpha-MEM medium containing 10% FBS, and incubated at 5% CO₂ and 37°C for 72 hours to confirm cell growth. After washed three times with PBS, serum components were removed as much as possible and the medium was replaced with a serum-free medium (Sigma C8730). After replaced with the serum-free medium, the culture medium was harvested every 24 hours, and a new serum-free medium was added. The culture medium was harvested for a total of 4 times and purified. After 200 ml of a blue sepharose resin (Amersham-Pharmacia) was filled in an XK50/20 column (Amersham-Pharmacia), 10 C.V. (column volume) of a buffer A (20 mM sodium phosphate, 1 M NaCl, pH 7.4) flowed to reach an equilibrium state. A sterilized and filtered culture solution flowed through a column in an equilibrium state at a flow rate of 20 ml/min, and monitored by connecting a UV detector with a wavelength of 280 nm. A buffer B (20 mM sodium phosphate, 1 M NaCl, 30% Ethylen Glycol, pH 7.4) flowed through the column to wash unadsorbed components, and the protein attached to the resin was eluted with a buffer C (20 mM sodium phosphate, 1 M NaCl, 60% Ethylen Glycol, pH 7.4). The eluate was dialyzed with phosphate buffered saline (PBS), concentrated with a concentrator (Centricon, Cut off 10,000), and dialyzed with phosphate buffered saline (PBS).

### 7. Comparison of 2 glycosylation purification efficiency of interferon-beta R27T variant and interferon-beta double variant (R27T and C17S)

In an interferon-beta variantABN 101 (NT) and an interferon-beta double variant ABN 101 (CS) purified using the blue sepharose resin, the content of a 2 glycosylated interferon-beta variant was measured using Reverse Phase High Performance Liquid Chromatography (RP-HPLC).

After each interferon variant was diluted to 0.5 mg/mL, acetonitrile (ACN) was mixed at an initial mobile phase ratio and then analyzed. RP-HPLC analysis was performed by a YMC-C4 column, and the solvents used therein were as follows. A mobile phase A was used after mixing 0.1% trifluoroacetic acid (TFA) with tertiary distilled water and then degassing for 1 hour after 0.2 µm PVDF filter. A mobile phase B was used after mixing 0.1% TFA with ACN and then degassing for about 1 hour after 0.2 µm PVDF filter.

The analysis conditions were indicated in the following Table.

**[Table 1]**

| Parameter | Condition |
|---|---|
| Column | TMC Pack C4, 4 x 250 mm, 5 µm |
| Column Temperature | 25°C |
| Flow Rate | 1.0 mL/min |
| Autosampler Temperature | 4°C |
| Wavelength | 214 nm or 280 nm |
| Injection Volume | 50 µl |

**[Table 2]**

| Time (min) | Eluent A (%) | Eluent B (%) |
|---|---|---|
| 0.0 | 70.0 | 30.0 |
| 1.0 | 70.0 | 30.0 |
| 18.0 | 45.0 | 55.0 |
| 18.1 | 0.0 | 100.0 |
| 22.0 | 0.0 | 100.0 |
| 22.1 | 70.0 | 30.0 |
| 30.3 | 70.0 | 30.0 |

### 8. Confirmation of interferon-beta variant stability according to buffer composition change

In each of an interferon-beta variant ABN 101 (NT) and an interferon-beta double variant ABN 101 (CS) purified using a blue sepharose resin, each buffer was replaced using a centricon. In order to confirm the degree of deterioration in quality due to aggregation occurring during buffer replacement of interferon drugs, each protein eluted from the Blue sepharose resin was obtained, and then concentration and buffer exchange were performed at a volume ratio of about 7 times using a 20 mM sodium phosphate (pH 2.9) buffer in the centricon. After the buffer exchange was completed with the centricon, the protein concentration was measured at a wavelength of 280 nm with a UV spectrophotometer after a 0.2 µm PES syringe filter to confirm the recovery rate.

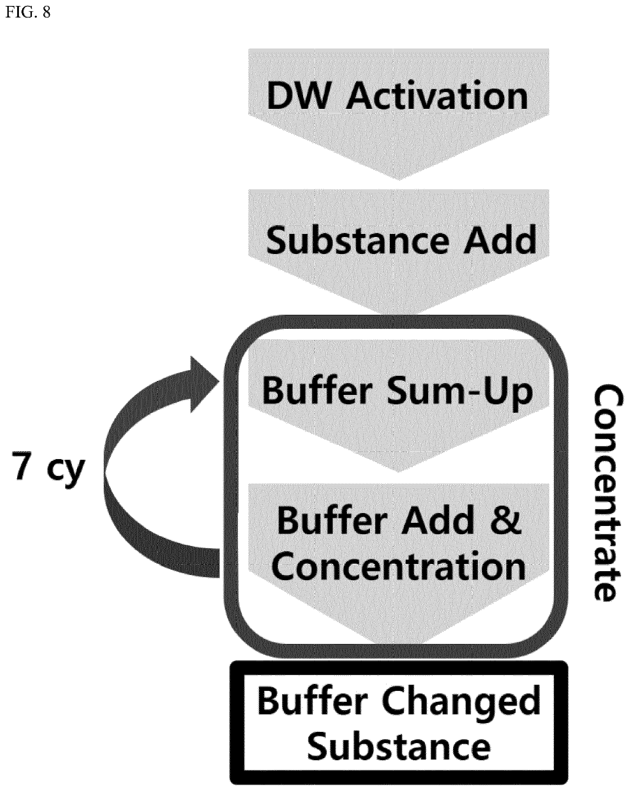

In addition, in order to confirm the storage stability with respect to other formulation buffers, the proteins that have been buffer-exchanged with 20 mM phosphate (pH 2.9) were again buffer-exchanged with 20 mM sodium acetate (pH 3.8) using a centricon at the same volume ratio level, respectively. The process was illustrated in FIG. 8.

### 9. Confirmation of freeze/thawing storage stability according to buffer composition change

To confirm freeze/thawing stability, for an interferon drug composed of each buffer, a freeze/thawing cycle of freezing at - 70°C for 12 hours or more and thawing at 25°C for 4 hours was performed 3 or 5 times and then Size Exclusion High Performance Liquid Chromatography (SEC-HPLC) analysis was performed. The storage stability of the interferon-beta variants was measured by confirming a ratio of the aggregation analyzed by a high molecular weight (HMWs) and the degradation analyzed by a low molecular weight (LMWs) to analyze a monomer change rate of the protein. For SEC-HPLC, a size exclusion column (TSKG2000) of Tosoh Co., Ltd. was used, and the solvents used were as follows. A mobile phase A was used by mixing tertiary distilled water and degassing for 1 hour after a 0.2 µm PVDF filter, and a mobile phase B was used by mixing 150 mM sodium chloride and 100 mM sodium phosphate dibasic dihydrate with tertiary distilled water and then titrating to pH 7.0 with phosphoric acid, and degassing after a 0.2 µm PVDF filter.

The analysis conditions were indicated in the following Table.

**[Table 3]**

| Parameter | Condition |
|---|---|
| Column | TSKgel G2000SKxL, 7.8 mm x 300 mm |
| Column Temperature | 25°C |
| Flow Rate | 0.5 mL/min |
| Autosampler Temperature | 4°C |
| Wavelength | 214 nm or 280 nm |
| Injection Volume | Sample : 50 µl |
| | GFS : 10 µl |

**[Table 4]**

| Time (min) | Eluent A (%) | Eluent B (%) |
|---|---|---|
| 0.0 | 0.0 | 100.0 |
| 40.0 | 0.0 | 100.0 |

### Results and Interpretation

### 1. Pool development result according to MSX concentration after gene transduction of ABN 101 (NT) and ABN 101 (CS)

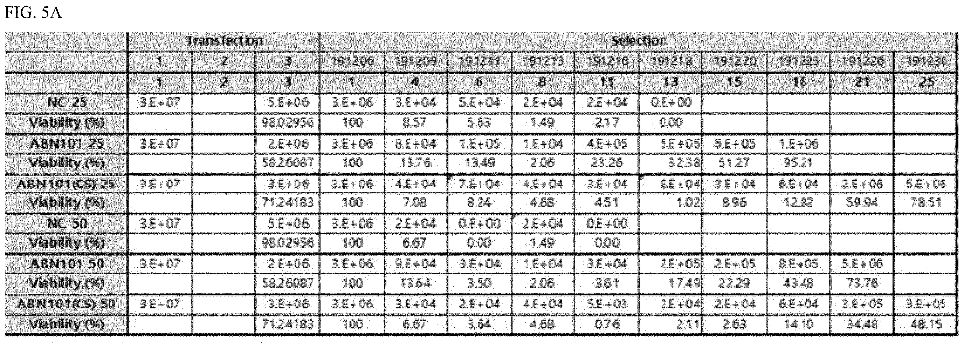

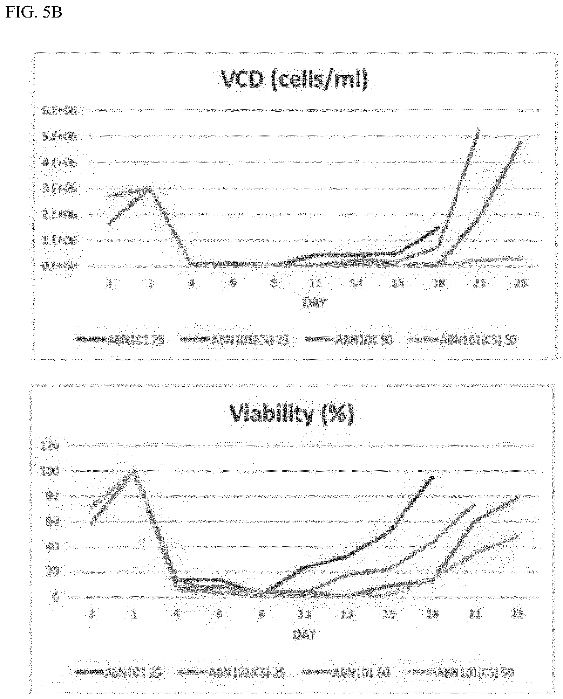

After CHO-K1 cells were transduced with DNA cloned into a pD2535nt-HDP vector, the number and viability of the cells were confirmed after 48 hours, and based on the number and viability of the cells, the selection of resistant cells with two concentrations of MSX was performed for 25 days. As a result, ABN 101(NT) obtained resistant cells earlier than ABN 101(CS), and in the case of 50 uM of ABN 101(CS), the final number of viable cells of 3 x 10⁵ /ml and the viability of 48% were shown, so that it was confirmed that the resistant cells were slowest obtained. Although the viability was low, it was determined that the growth and viability were affected according to the expression of interferon beta-1a. The results thereof were illustrated in FIGS. 5A and 5B.

### 2. Fed-batch results in ABN 101 (NT) and ABN 101 (CS) pool states

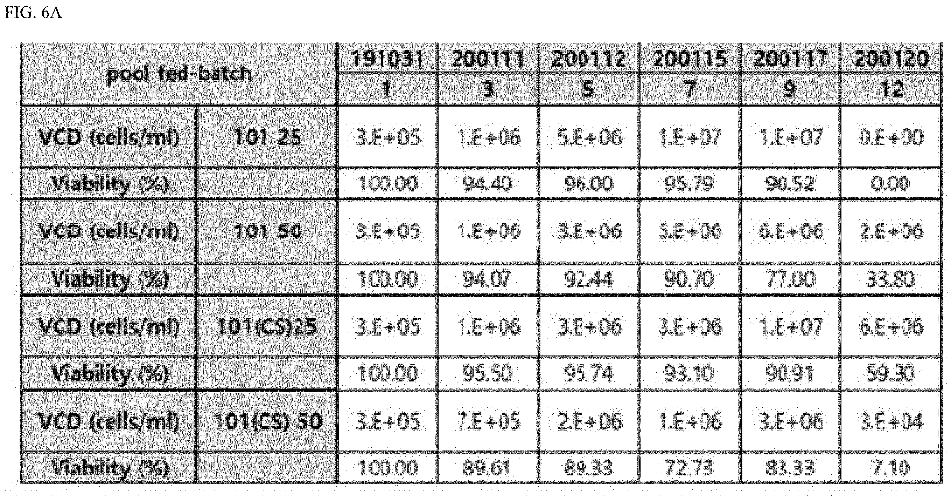

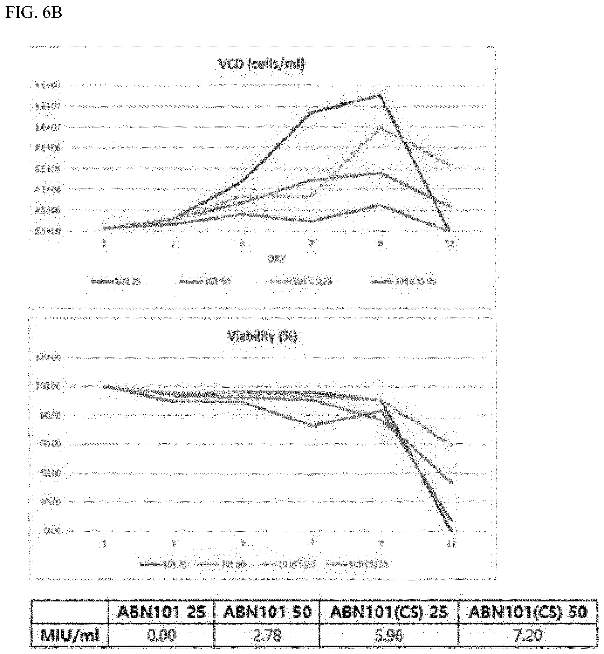

The results of performing 50 ml small scale Fed-batch with cells in a pool state were as follows. In both the ABN 101 (NT) pool and the ABN 101 (CS) pool, it was confirmed that the viability was maintained longer in the pool with the MSX concentration of 50 µM than 25 µM, and it was confirmed that the number of viable cells was less maintained in the pool with the MSX concentration of 50 µM than 25 µM. However, although the number of viable cells was less maintained in the pool with the MSX concentration of 50 µM than 25 µM, the biological activity of Interferon beta-1a in the culture medium on the last day of Fed-batch was higher and it was confirmed that the biological activity of the ABN 101(CS) pool was at least 2 times higher than that of the ABN 101(NT) pool. The results were illustrated in FIGS. 6A and 6B.

### 3. 1L scale fed-batch results of ABN 101 (NT) and ABN 101 (CS) pools

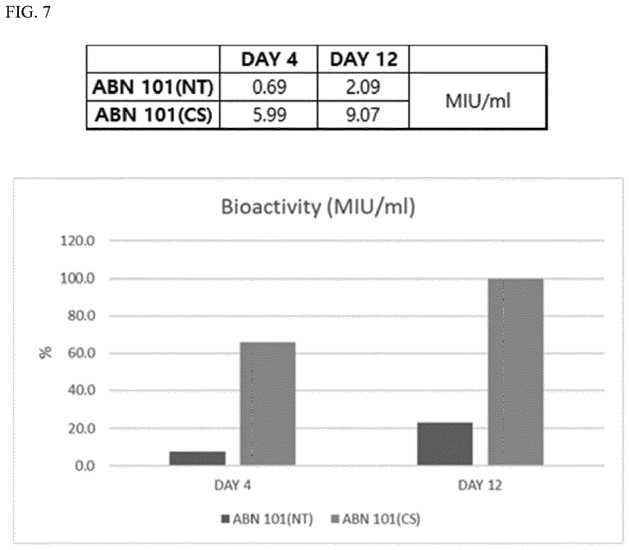

Based on the small scale fed-batch results above, 1L scale fed-batch was performed using a pool with a MSX concentration of 50 µM. As a result, the biological activity on the 4th day of culture was 0.69 MIU/ml for ABN 101(NT) and 5.99 MI/ml for ABN 101(CS), so that it was confirmed that the biological activity of ABN 101(CS) was about 8.6 times higher. When comparing the biological activity of the 12th day of culture, it was confirmed that the biological activity of ABN 101 (CS) was about 4.5 times higher. The results were illustrated in FIG. 7.

### 4. Purification result of interferon-beta variant

As a result of the purification of the interferon-beta variant, the purification efficiency was higher than that of conventional human interferon-beta. As a result, it was confirmed that a human interferon-beta variant comprising an amino acid sequence having serine substituted for the 17th amino acid cysteine and threonine substituted for the 27th amino acid arginine had higher purification efficiency.

### 5. Result of 2 glycosylation purification rate of interferon-beta R27T variant and interferon-beta double variant (R27T and C17S)

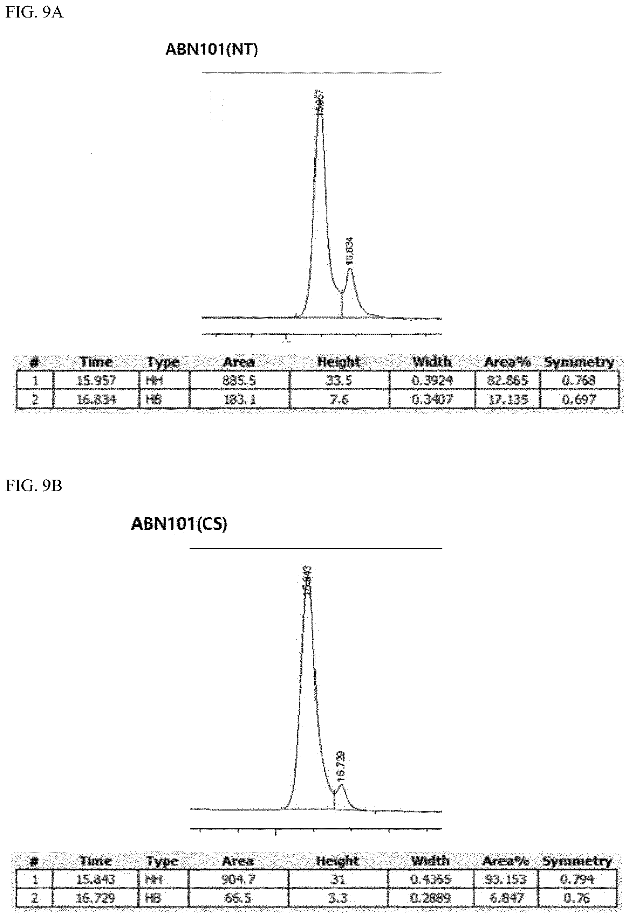

The interferon-beta variants purified using a blue sepharose resin were analyzed with the content of 2 glycosylation: 1 glycosylation, respectively. As a result, as shown in Table 5 and FIGS. 9A and 9B below, it was confirmed that the 2 glycosylation content of the ABN 101 (CS) double variant was about 10% higher than that of ABN 101 (NT). There was no significant difference when comparing the biological activities of each material through ELISA, but it was confirmed that the purification efficiency of 2 glycosylated protein was high when the interferon-beta variant was expressed and purified on the same scale.

**[Table 5]**

| Material | 2 glycosylation | 1 glycosylation | MIU/mg |
|---|---|---|---|
| ABN 101(NT) | 82.8 | 17.1 | 351 |
| ABN 101(CS) | 93.1 | 6.9 | 360 |

### 6. Result of interferon-beta variant stability according to buffer composition change

In order to confirm the stability of the interferon-beta variant for each buffer composition, protein recovery rate and SEC-HPLC analysis were performed by exchanging each buffer with a centricon. When each protein was purified and then the buffer was exchanged with 20 mM sodium phosphate (pH 2.9), it was confirmed that the recovery rate thereof was high in ABN 101 (CS) as shown in Table 6 below.

**[Table 6]**

| Material | Initial Conc.(mg/mL) | 20 mM Na-Pi (mg/mL) | Recovery (%) |
|---|---|---|---|
| ABN 101(NT) | 0.56 (30mL) | 1.1 (8mL) | 52 |
| ABN 101(CS) | 0.40 (15mL) | 0.7 (8mL) | 93 |

In addition, the recovery rate was confirmed by replacing the buffer with 20 mM sodium acetate (pH 3.8) using a centricon in the same manner to replace each protein exchanged with the corresponding buffer with a buffer based on a different formulation again. As a result, it was confirmed that the recovery rate of ABN 101 (CS) was also higher than that of ABN 101 (NT) when exchanged with an acetate-based buffer as shown in Table 7. This was interpreted as protein structural stability from physical factors such as a centricon by increased stability by the double variant.

**[Table 7]**

| Material | Initial Conc. (mg/mL) | 20 mM Na-OAc (mg/mL) | Recovery (%) |
|---|---|---|---|
| ABN 101(NT) | 1.1 (1mL) | 0.86 (1mL) | 78 |
| ABN 101(CS) | 1.7 (1. 1mL) | 1.7 (1mL) | 91 |

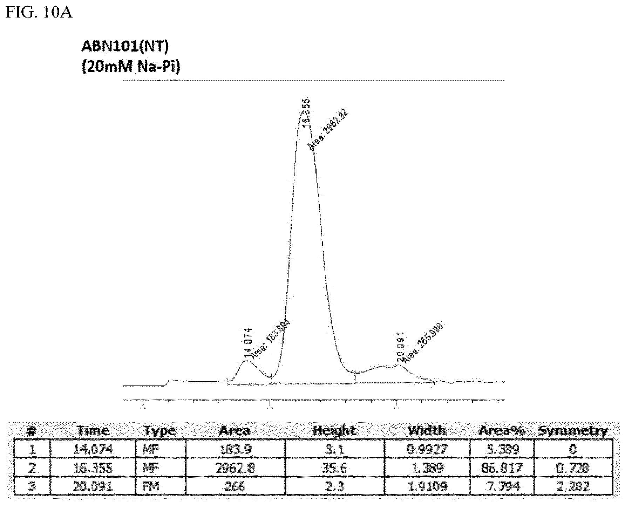

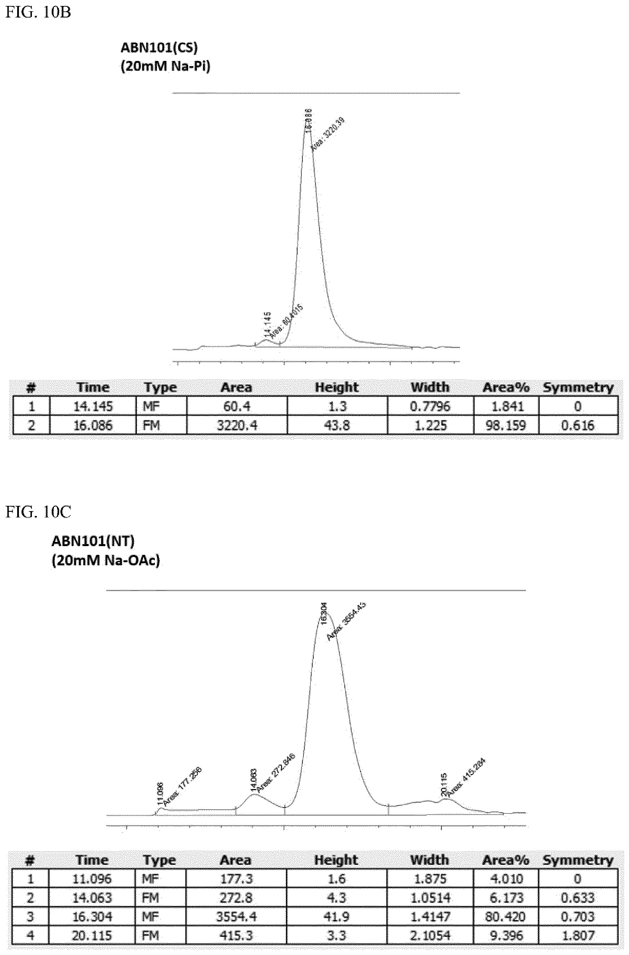

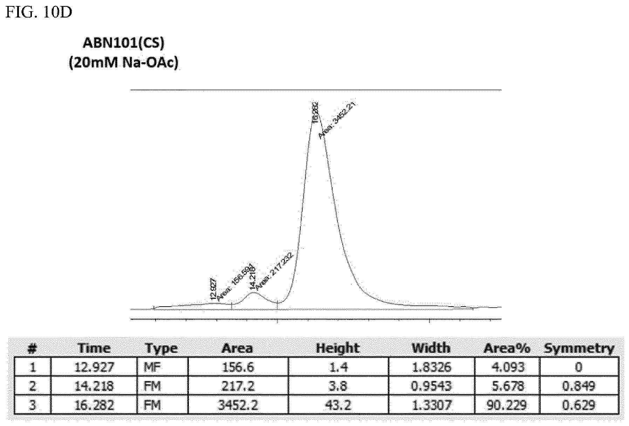

The protein recovery rate and the monomer analysis result for each buffer composition change were shown in Table 8 and FIGS. 10A to 10D below.

**[Table 8]**

| Material | 20 mM Na-Pi (Monomer %) | 20 mM Na-OAc (Monomer %) | Monomer Recovery (%) |
|---|---|---|---|
| ABN 101(NT) | 86.8% | 80.4% | 92.6 |
| ABN 101(CS) | 98.1% | 90.2% | 91.8 |

### 7. Result of Freeze/thawing stability according to buffer composition change

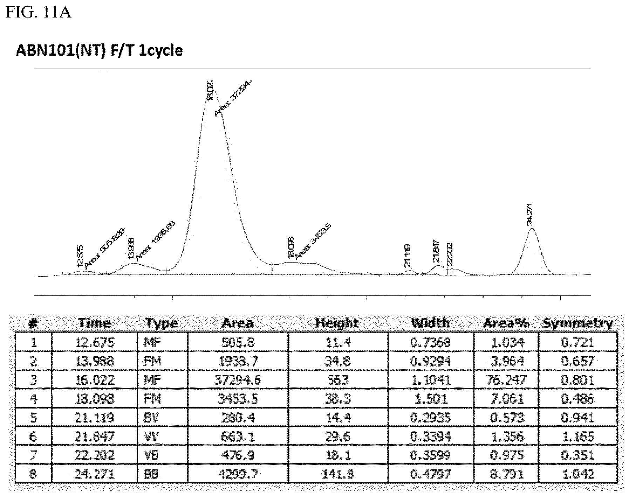

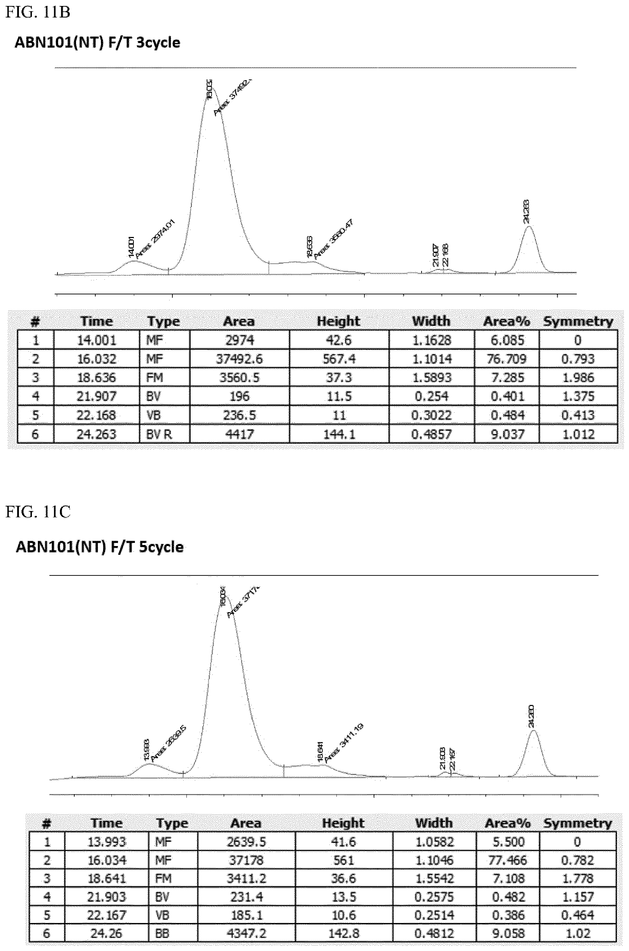

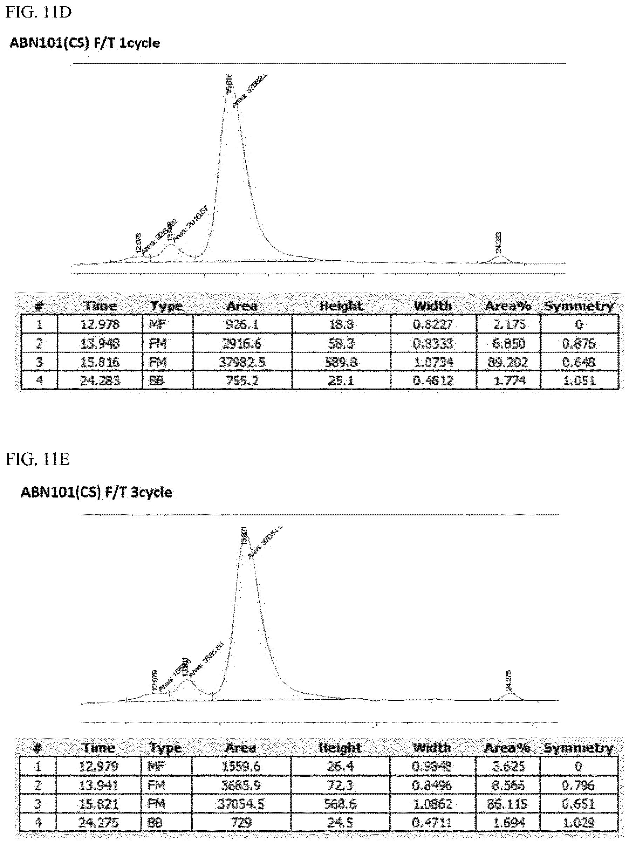

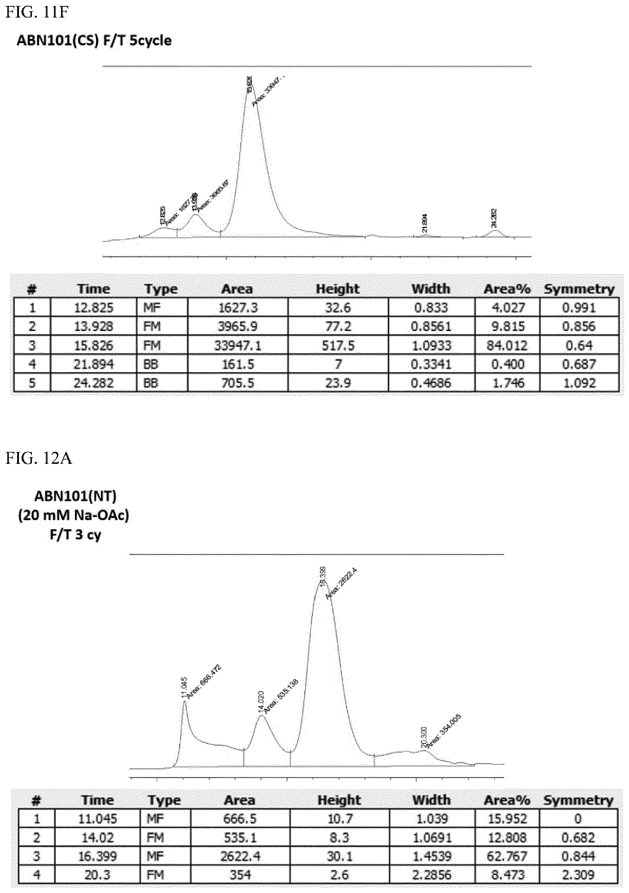

In order to confirm the freeze/thawing stability of the interferon-beta variant by buffer composition, the interferon-variant in the form of a substituted buffer was repeatedly freeze-thawed 3 times or 5 times, respectively, and the monomer change rate was confirmed through SEC-HPLC analysis. As a result, when the interferon-variants were stored in a buffer based on 20 mM sodium phosphate (pH 2.9) and subjected to repeated freeze-thawing, a difference between the two interferon-beta variants was not significant (see Table 9, Table 10, and FIGS. 11A to 11F).

**[Table 9]**

| ABN 101(NT) | HMWs (%) | Monomers (%) | LMWs (%) |
|---|---|---|---|
| F/T 1cy | 5 | 76 | 19 |
| F/T 3cy | 6 | 76 | 18 |
| F/T 5cy | 5 | 77 | 18 |

**[Table 10]**

| ABN 101(CS) | HMWs (%) | Monomers (%) | LMWs (%) |
|---|---|---|---|
| F/T 1cy | 8 | 89 | 3 |
| F/T 3cy | 11 | 86 | 3 |
| F/T 5cy | 13 | 84 | 3 |

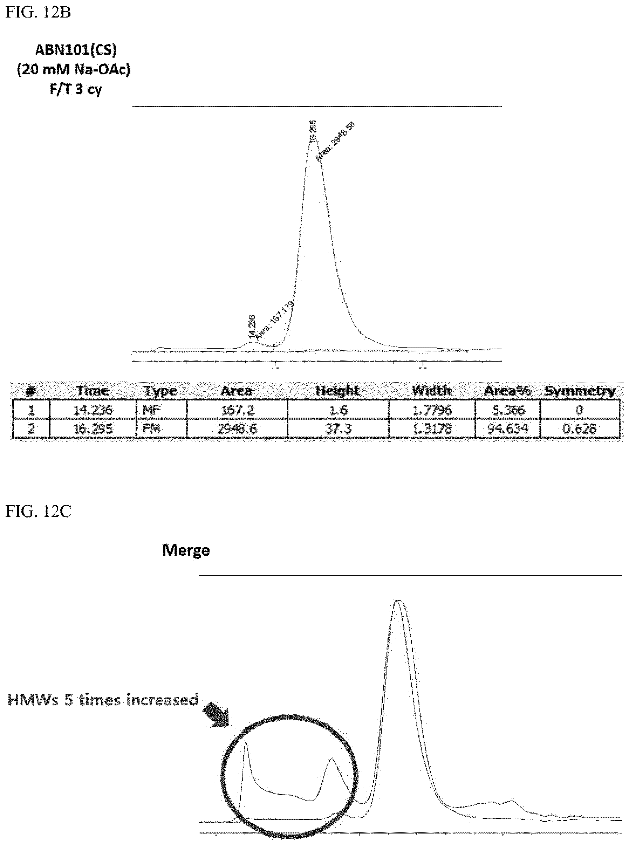

However, as a result analyzed by exchanging the buffer with 20 mM sodium acetate (pH 3.8) and performing a freeze-thaw test, ABN 101 (CS) maintained the monomer content, but ABN 101 (NT) showed rapidly increased HMWs compared to ABN 101 (CS) (5-fold increased) (see Table 11, Table 12, and FIGS. 12A to 12C). Through these results, it can be expected that the storage instability of the acetate-based interferon-variant drug may be compensated due to the stability-increasing effect of the double variant of ABN 101(CS).

**[Table 11]**

| ABN 101(NT) (20 mM Na-OAc) | HMWs (%) | Monomers (%) | LMWs (%) |
|---|---|---|---|
| Origin | 10 | 80 | < 10 |
| F/T 3cy | 28 | 63 | <9 |

**[Table 12]**

| ABN 101(CS) (20 mM Na-OAc) | HMWs (%) | Monomers (%) | LMWs (%) |
|---|---|---|---|
| Origin | 9 | 90 | < 1 |
| F/T 3cy | 5 | 94 | < 1 |

### INDUSTRIAL APPLICABILITY

Therefore, the present invention provides a human interferon-beta variant comprising an amino acid sequence having threonine substituted for the 27th amino acid arginine and serine substituted for the 17th amino acid cysteine of human interferon-beta. The present invention provides a human interferon-beta variant with improved purification efficiency, which can be usefully used in the production of a therapeutic agent using the same, and thus has excellent industrial applicability.

## Claims

1. A human interferon-beta variant comprising an amino acid sequence having serine substituted for the 17th amino acid cysteine and threonine substituted for the 27th amino acid arginine of human interferon-beta.

2. A polynucleotide encoding the human interferon-beta variant of claim 1.

3. The polynucleotide of claim 2, wherein the human interferon-beta variant is a human interferon-beta variant comprising an amino acid sequence of SEQ ID NO: 3.

4. An expression vector expressing human interferon-beta in an animal cell comprising the polynucleotide of claim 2.

5. An animal cell transformed with the expression vector of claim 4.

6. A method for preparing a human interferon-beta variant comprising culturing the animal cell of claim 5.

7. A pharmaceutical composition comprising the human interferon-beta variant of claim 1 as an active ingredient, wherein the pharmaceutical composition has a pharmaceutical effect of natural human interferon-beta.

8. The pharmaceutical composition of claim 7, wherein the pharmaceutical composition has a pharmaceutical effect of natural human interferon-beta, and the pharmaceutical effect is a pharmaceutical effect of preventing or treating a disease selected from the group consisting of multiple sclerosis, cancer, autoimmune disorder, viral infection, HIV-related diseases, and hepatitis C.

9. A method for improving stability of a human interferon-beta R27T variant, comprising substituting serine for the 17th amino acid cysteine in the human interferon-beta R27T variant in which threonine is substituted for the 27th amino acid arginine of human interferon-beta.

10. The method of claim 9, wherein the human interferon-beta R27T variant consists of an amino acid sequence of SEQ ID NO: 2.

11. The method of claim 9, wherein the stability is selected from the group consisting of purification stability, storage stability and freeze/thawing stability.

12. The method of claim 11, wherein the purification stability improves purification efficiency of 2 glycosylation proteins.

13. The method of claim 11, wherein the purification stability reduces the protein aggregation and degradation during concentration and buffer exchange.

14. The method of claim 11, wherein the storage stability is storage stability in a buffer of pH 2.0 to 6.0.

15. The method of claim 14, wherein the buffer is a buffer selected from the group consisting of acetic acid, phosphoric acid, ammonium carbonate, ammonium phosphate, boric acid, citric acid, lactic acid, potassium citrate, potassium metaphosphate, potassium phosphate monobasic, sodium acetate, sodium citrate, sodium lactate solution, dibasic sodium phosphate, monobasic sodium phosphate, bicarbonate, tris(tris(hydroxymethyl)aminomethane), 3-(N-morpholino)propanesulfonic acid (MOPS), N-(2-hydroxyethyl)piperazine-N'-(2-ethanesulfonic acid) (HEPES), 2-(2-amino-2-oxoethyl)aminoethanesulfonic acid (ACES), N-(2-acetamido)2-iminodiacetic acid (ADA), 3-(1,1-dimethyl-1,2-hydroxyethylamino-2-propanesulfonic acid (AMPSO), N,N-bis(2-hydroxyethyl)-2-aminoethanesulfonic acid (BES), N,N-bis(2-hydroxyethylglycine (Bicine), bis-tris (bis-(2-hydroxyethyl)imino-tris(hydroxymethyl)methane, 3-(cyclohexylamino)-1-propanesulfonic acid (CAPS), 3-(cyclohexylamino)-2-hydroxy-1-propanesulfonic acid (CAPSO), 2-(N-cyclohexylamino)ethanesulfonic acid (CHES), 3-N,N-bis(2-hydroxyethylamino-2-hydroxy-propanesulfonic acid (DIPSO), N-(2-hydroxyethylpiperazine)-N'-(3-propanesulfonic acid) (HEPPS), N-(2-hydroxyethyl)piperazine-N'-(2-hydroxypropanesulfonic acid (HEPPSO), 2-(N-morpholino)ethanesulfonic acid (MES), triethanolamine, imidazole, glycine, ethanolamine, phosphate, 3-(N-morpholino)-2-hydroxypropanesulfonic acid (MOPSO), piperazine-N,N'-bis(2-ethanesulfonic acid (PIPES), piperazine-N,N'-bis(2-hydroxypropanesulfonic acid (POPSO), N-trishydroxymethyl)methyl-3-aminopropanesulfonic acid (TAPS), 3-N-tris(hydroxymethyl)methylamino-2-hydro hydroxy-propanesulfonic acid (TAPSO), N-tris(hydroxymethyl)methyl-2-aminoethanesulfonic acid (TES), N-tris(hydroxymethyl)methylglycine (Tricine), 2-amino-2-methyl-1,3-propanediol, and 2-amino-2-m ethyl-1 -propanol.

16. The method of claim 11, wherein the freeze/thawing stability is thawing stability after freezing at - 100°C to - 10°C.

17. The method of claim 11, wherein the freeze/thawing stability is freeze/thawing stability in an acetic acid buffer.

18. The method of claim 11, wherein the freeze/thawing stability reduces the protein aggregation and degradation after 3 times or more freeze/thawing cycles.

19. Use of the human interferon-beta variant of claim 1 for preparing an agent having a pharmaceutical effect of natural human interferon-beta on a disease selected from the group consisting of multiple sclerosis, cancer, autoimmune disorders, viral infection, HIV-related diseases, and hepatitis C.

20. A method for treating a disease selected from the group consisting of multiple sclerosis, cancer, autoimmune disorders, viral infection, HIV-related diseases, and hepatitis C, comprising administering an effective amount of a composition having a pharmaceutical effect of natural human interferon-beta and comprising the human interferon-beta variant of claim 1 to a subject in need thereof.

21. A human interferon-beta variant having serine substituted for the 17th amino acid cysteine and threonine substituted for the 27th amino acid arginine of human interferon-beta of SEQ ID NO: 1, having at least 90% sequence homology with wild-type interferon beta of SEQ ID NO: 1, and having the activity of interferon beta.
